Europäisches Patentamt

European Patent Office

Office européen des brevets

(19)

(11) Veröffentlichungsnummer: **0 291 804**
**A2**

(12) **EUROPÄISCHE PATENTANMELDUNG**

(21) Anmeldenummer: 88107401.7

(22) Anmeldetag: 07.05.88

(51) Int. Cl.⁴: **C07K 13/00** , **C12P 21/02** ,
**A61K 37/02**

(30) Priorität: 16.05.87 DE 3716513

(43) Veröffentlichungstag der Anmeldung:
23.11.88 Patentblatt 88/47

(84) Benannte Vertragsstaaten:
**AT BE CH DE ES FR GB IT LI NL SE**

(71) Anmelder: **BASF Aktiengesellschaft**
**Carl-Bosch-Strasse 38**
**D-6700 Ludwigshafen(DE)**

(72) Erfinder: **Doerper, Thomas, Dr.**
**Luitpoldstrasse 3**
**D-6719 Bissersheim(DE)**
Erfinder: **Moeller, Achim, Dr.**
**Wilhelm-Busch-Strasse 51**
**D-6703 Limburgerhof(DE)**
Erfinder: **Hillen, Heinz, Dr.**
**Fasanenstrasse 10**
**D-6700 Ludwigshafen(DE)**
Erfinder: **Keilhauer, Gerhard, Dr.**
**Industriestrasse 20**
**D-6701 Dannstadt-Schauernheim(DE)**
Erfinder: **Emling, Franz, Dr.**
**Valentin-Bauer-Strasse 22**
**D-6700 Ludwigshafen(DE)**
Erfinder: **Lothar, Daum, Dr.**
**Reiherstrasse 25**
**D-6701 Otterstadt(DE)**

(54) Proteine mit TNF-Wirkung.

(57) Es werden Derivate des Tumor-Nekrose-Faktors (TNF), die durch eine Veränderung am Aminoterminus aus dem TNF-Molekül hervorgehen, sowie deren Herstellung beschrieben. Die Proteine eignen sich zur Bekämpfung von Krankheiten.

EP 0 291 804 A2

0 291 804

## Proteine mit TNF-Wirkung

Die Erfindung betrifft neue Proteine mit TNF-Wirkung, deren Herstellung und deren Verwendung in der Therapie.

Von Carswell et al. [Proc. Natl. Acad. Sci. USA 72,3666-3670, 1975] wurde berichtet, daß das Serum von Endotoxin-behandelten Tieren, die zuvor mit Mycobacterien-Stamm Calmette-Guerin (BCG) infiziert worden waren, eine hämorrhagische Nekrose bei verschiedenen Tumoren in der Maus bewirkte. Diese Aktivität wurde einem Tumor-Nekrose-Faktor (TNF) zugeschrieben. TNF zeigt auch eine cytostatische oder cytotoxische Wirkung gegenüber einer Vielzahl von transformierten Zellinien in vitro, während normale menschliche und tierische Zellinien davon nicht betroffen werden [Ruff und Gifford, Lymphokine Reports Vol. 2, pp 235-275, Academic Press, New York, 1981]. Kürzlich wurde die biochemische Charakterisierung und das Gen für den menschlichen TNF beschrieben [Aggarwal et al., J. Biol. Chem. 260, 245-2354, 1985; Nedwin et al., Nucl. Acids Res. 13, 6361-673, 1985]. Aus diesen Daten läßt sich folgende Proteinstruktur für das reife humane TNF ableiten:

ValArgSerSerSerArgThrProSerAspLysProValAlaHisValValAlaAsnPro    GlnAlaGluGlyGlnLeuGlnTrpLeuAsnArggArgAlaAsnAlaLeuLeuAlaAsnGly  ValGluLeuArgAspAsnGlnLeuValValProSerGluGlyLeuTyrLeuIleTyrSer  GlnValLeuPheLysGlyGlnGlyCysProSerThrHisValLeuLeuThrHisThrIle    SerArgIleAlaValSerTyrGlnThrLysValAsnLeuLeuSerAlaIleLysSerPro  CysGlnArgGluThrProGluGlyAlaGluAlaLysProTrpTyrGluProIleTyrLeu  GlyGlyValPheGlnLeuGluLysGlyAspArgLeuSerAlaGluIleAsnArgProAspTyrLeuAspPheAlaGluSerGlyGlnValTyrPheGlyIleIleAlaLeu

Fibronectin ist ein im menschlichen Plasma vorkommendes Glykoprotein mit einem Molekulargewicht von etwa 450.000. Es besteht aus zwei Disulfid-verknüpften Polypeptidketten, die eine Zell-bindende Domäne enthalten [Hynes et al., J. Cell Biol. 95, 369-377, 1982]. Die Primärstruktur dieser Zell-bindenden Domäne wurde von Pierschbacher et al., J. Biol. Chem. 257, 9593-9597, 1982, ermittelt. Mit dieser Domäne kann das Fibronectinmolekül an einen Rezeptor auf der Oberfläche von Zellen wie Blutplättchen oder Fibroblasten binden [Plow und Ginsberg, J. Biol. Chem. 256, 9477-9482, 1981].

Fügt man Partialsequenzen aus der Zell-bindenden Domäne des menschlichen Fibronectins an den Aminoterminus des reifen humanen TNF so erhält man Hybridproteine, die vorteilhaftere Eigenschaften besitzen als das humane TNF selbst.

Die Erfindung betrifft Proteine mit folgender Aminosäuresequenz:

$Y$-Val-$X$-(Ser)$_n$-ArgThrProSerAspLysProValAlaHisValValAlaAsnPro  GlnAlaGluGlyGlnLeuGlnTrpLeuAsnArggAlaAsnAlaLeuLeuAlaAsnGly  ValGluLeuArgAspAsnGlnLeuValValProSerGluGlyLeuTyrLeuIleTyrSer  GlnValLeuPheLysGlyGlnGlyCysProSerThrHisValLeuLeuThrHisThrIle  SerArgIleAlaValSerTyrGlnThrLysValAsnLeuLeuSerAlaIleLysSerPro    CysGlnArgGluThrProGluGlyAlaGluAlaLysProTrpTyrGluProIleTyrLeu  GlyGlyValPheGlnLeuGluLysGlyAspArgLeuSerAlaGluIleAsnArgProAsp  TyrLeuAspPheAlaGluSerGlyGlnValTyrPheGlyIleIleAlaLeu,

worin

$n$    0, 1, 2, 3 oder 4,

$X$    eine Teilsequenz aus der Zell-bindenden Domäne des Fibronectin-Moleküls und

$Y$    ein Wasserstoffatom oder ein Methioninrest sind.

Unter diesen Proteinen sind diejenigen bevorzugt, in denen $n$ 1, 2, 3 oder 4, $X$ eine Teilsequenz aus 10-20 Aminosäuren und $Y$ ein Wasserstoffatom ist.

Die neuen Proteine lassen sich herstellen, indem man

    a. einen Vektor herstellt, der die genetische Information für die Proteine gemäß Anspruch 1 enthält,

    b. den Vektor vermehrt

    c. die für Expression nötigen Signale in den Vektor einbaut,

    d. den Vektor in einen Wirtsorganismus einbringt und

    e. den Wirtsorganismus vermehrt und das Protein isoliert.

Zur Herstellung eines geeigneten Vektors geht man von der entsprechenden cDNA aus.

Für die Isolierung der entsprechenden cDNA wurde die Monocyten-Zellinie HL 60 (ATCC Nr. CCL 240) wie beschrieben kultiviert (Pennica et al., Nature 312, 724-729, 1984), die mRNA nach Stimulierung isoliert und nach bekannten Verfahren in cDNA übersetzt.

Durch Einsetzen dieser cDNA in den kommerziell erhältlichen Klonierungsvektor Lambda gt 10 wurde eine cDNA-Bibliothek angelegt.

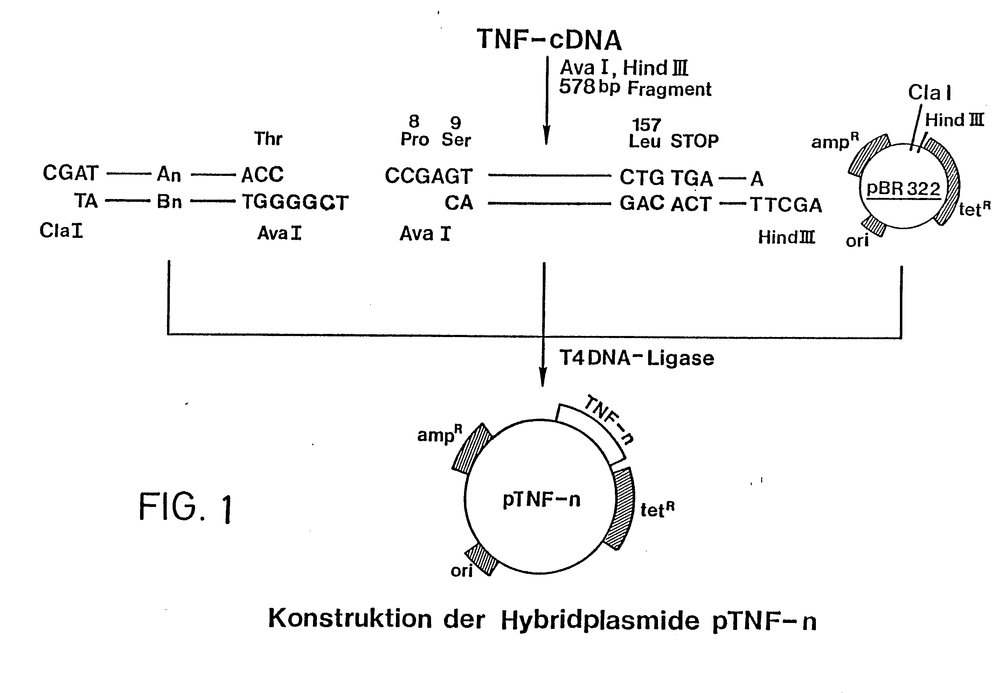

Mit Hilfe von radioaktiv markierten Oligonukleotidsonden wurde ein cDNA-Klon identifiziert, der den kodierenden Teil des TNF-Gens enthält (Fig. 1).

2

Teile dieser Sequenz, die durch Restriktionserkennungsstellen leicht zugänglich sind, werden benutzt, um die in den Beispielen näher beschriebenen neuen TNF-Hybridgene zu klonieren (Fig. 1). Der Einbau der Genfragmente in Klonierungsvektoren, beispielsweise in die handelsüblichen Plasmide pBR 322 und pBR 327 erfolgte in an sich bekannter Weise. Auch können die Gene oder Genfragmente mit geeigneten chemisch synthetisierten Kontrollregionen versehen werden, die eine Expression der Proteine ermöglichen. Die Transformation der so erhaltenen Hybridplasmide in geeignete Wirtsorganismen, vorteilhaft E. coli, ist ebenfalls bekannt und eingehend beschrieben. Auch können die Hybridplasmide mit entsprechenden Signalsequenzen versehen werden, die die Sektretion der Polypeptide in das Periplasma von E. coli erlauben.

Aufgrund der Degeneration des genetischen Codes ist es aber auch möglich, andere DNA-Sequenzen, z.B. chemisch synthetisierte TNF-Gene mit unterschiedlicher DNA-Sequenz, für die Expression von TNF-Hybridgenen zu benutzen.

Die neuen Hybridproteine mit TNF-Wirkung können als neue Wirkstoffe in der Therapie von malignen Erkrankungen des Menschen eingesetzt werden.

Beispiele

Herstellung von Plasmiden

1. Herstellung eines Hybridplasmids, das das Genfragment für ein TNF-Derivat mit verändertem Amino-Terminus trägt.

Ausgangsmaterial ist ein rekombinanter Phage mit der cDNA von TNF der nach dem von Pennica et al. (Nature 312,724-729, 1984) beschriebenen Verfahren erhalten wurde. Dazu wurde die menschliche Monocyten-Zellinie HL 60 (ATCC CCL240) mit Phorbolester (PMA) behandelt, um sie zur TNF-Produktion anzuregen. 4 h nach der Phorbolesterbehandlung wurde die RNA aus dieser Zellinie isoliert und daraus nach Maniatis: Molecular Cloning, Cold Spring Harbor Laboratory, 1982, Seiten 224ff die cDNA hergestellt. Mit dieser cDNA wurde eine Genbank angelegt, wobei als Vektor der Lambda-Phage gt10 benutzt wurde. Mit Hilfe einer TNF-spezifischen, chemisch synthetisierten Oligonukleotidprobe, die radioaktiv markiert war, wurde die Genbank durchgemustert. Von einem dabei als positiv identifizierten Klon wurde durch Spaltung mit den Restriktionsenzymen Ava1 und Hind3 ein 578bp langes DNA-Fragment erhalten, das für die Aminosäuren 8 bis 157 des humanen TNF-Moleküls codiert. Dieses Fragment wurde durch Elektrophorese in einem 1 % Agarosegel von den anderen entstandenen Fragmenten abgetrennt und aus dem Gel nach bekannten Verfahren eluiert [Maniatis et al., Cold Spring Harbor Laboratory, Seite 164, 1982]. Das erhaltene Bruchstück wurde anschließend in einen Vektor eingebaut, der durch Spaltung des Plasmids pBR322 mit den Restriktionsendonukleasen Cla1 und Hind3 erhalten worden war. Das bei der Spaltung entstandene große Fragment wurde durch eine zweimalige Ethanolfällung rein erhalten.

Als Cla-Ava-Adaptor diente ein äquimolares Gemisch synthetisch hergestellter Oligonukleotide A1 und B1, die folgende Primärstruktur besitzen:

```
A1:  5'-CGATACTACTATGGTCAGATCTTCATCTTCTCGAACC          -3'
B1:  3'-  TATGATGATACCAGTCTAGAAGTAGAAGAGCTTGGGGCT     -5'
```

Man erhielt das neue Hybridplasmid pTNF-1, indem man 0,1 pMol des Vektorfragments, 0,2 pMol des 578 bp langen TNF-Fragments und jeweils 0,5 pMol A1 und B1 mit dem Enzym T4-DNA-Ligase verknüpfte (Fig. 1, An und Bn bedeuten darin die Oligonukleotide A1, A2 ... und B1, B2 ...)

3

0 291 804

2. TNF-Derivate mit verändertem Aminoterminus

Die Konstruktion erfolgte analog Beispiel 1 mit den neuen Oligonukleotiden A2/B2, A3/B3, A4/B4 und A5/B5:

```
A2: 5'-CGATACTACTATGGTCAGATCTAGATCATCTTCTTCGCGAACC        -3'
B2: 3'-  TATGATGATACCAGTCTAGATCTAGTAGAAGAAGCGCTTGGGGCT  -5'


A3: 5'-CGATACTACCATGGTCTACGCTGTCACCGGCCGTGGTGACTCTCCTGCTTCA-
B3: 3'-  TATGATGCTACCAGATGCGACAGTGGCCGGCACCACTGAGAGGACGAAGT
     TCTTCTCGAACC       -3'
     AGAAGAGCTTGGGGCT  -5'


A4: 5'-CGATACTACCATGGTCTACGCTGTCACCGGCCGTGGTGACTCTCCTATCGACGGTCGA
B4: 3'-  TATGATGGTACCAGATGCGACAGTGGCCGGCACCACTGAGAGGATAGCTGCCAGCT
     ACC-3'
     TGGGGCT-5'
```

Man erhielt jeweils die neuen Hybridplasmide pTNF-2, pTNF-3 und pTNF-4.


3. Herstellung von Hybridplasmiden, die die Genfragmente für Fibronectin-TNF-Hybridproteine tragen

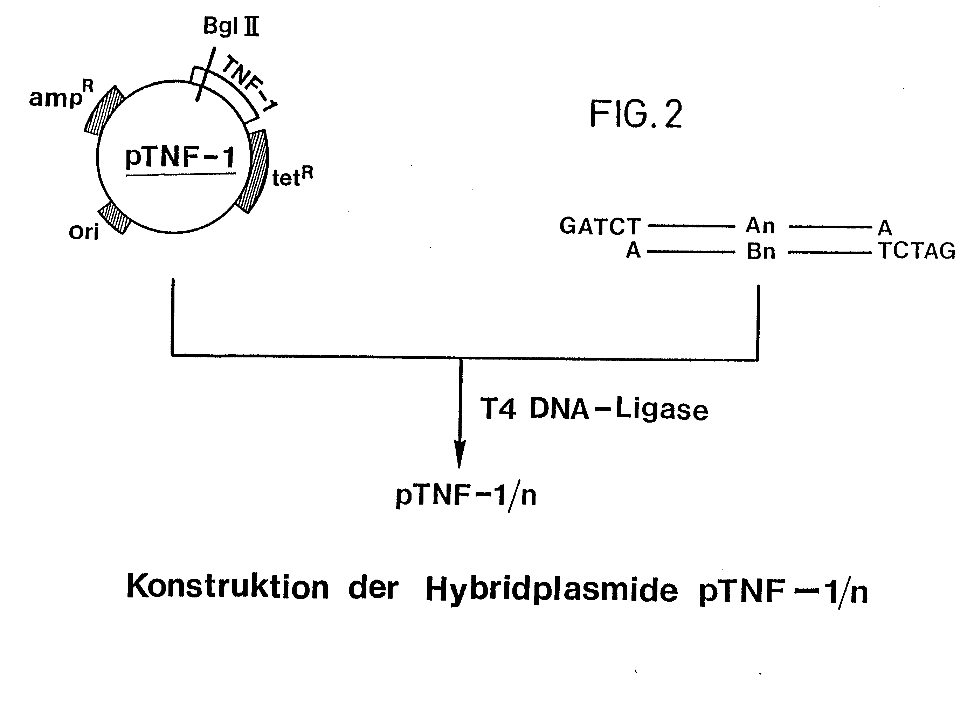

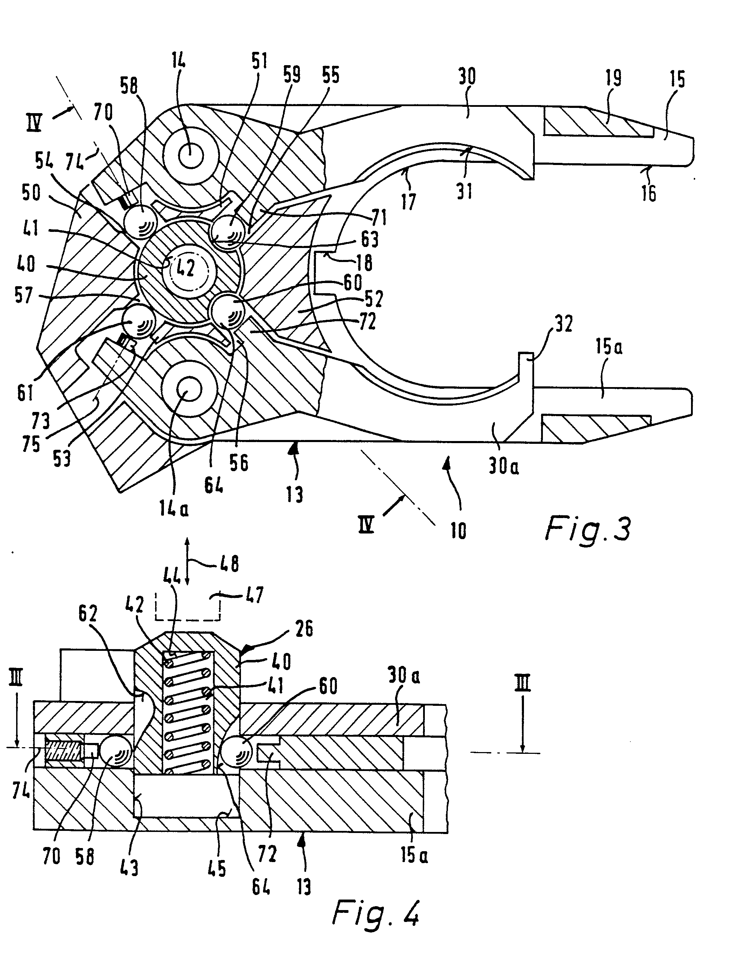

Ausgangspunkt ist das in Beispiel 1 konstruierte Plasmid pTNF-1. Dieses trägt eine singuläre Bgl2-Erkennungsstelle (AGATCT). Das Plasmid pTNF-1 wurde mit dem Restriktionsenzym Bgl2 geöffnet. Zu 0,1 pMol dieses DNA-Fragments wurden 0,3 pMol eines äquimolaren Gemisches von synthetisch hergestellten A5 und B5 hinzugefügt und durch eine durch T4-DNA-Ligase katalysierte Reaktion verknüpft (Fig. 2).

```
A5: 5'-GATCTGTCACCGGCCGTGGTGACTCTCCTGCTA        -3'
B5: 3'-    ACAGTGGCCGGCACCACTGAGAGGACGATCTAG    -5'
```

Man erhielt das neue Hybridplasmid pTNF-1/5. Bei dieser Konstruktion kann das A5/B5-Fragment in beliebiger Orientierung in den Vektor eingebaut werden. Deshalb wurden zwei verschiedene Hybridplasmide erhalten, die sich bezüglich der Orientierung des A5/B5-Fragments unterscheiden und bei anschließender Genexpression verschiedene Hybridproteine produzieren. Die Orientierung läßt sich mit bekannten DNA-Sequenzierungsverfahren bestimmen.


4. Herstellung von Hybridplasmiden, die Genfragmente für weitere Fibronectin-TNF-Hybridproteine tragen

Die Konstruktion erfolgt analog Beispiel 3 mit den neuen Oligonukleotidgemischen A6/B6 und A7/B7:

```
A6: 5'-GATCTTACGCTGTCACCGGCCGTGGTGACTCTCCTGCTAGCTCAAAGCCTA        -3'
B6: 3'-    AATGCGACAGTGGCCGGCACCACTGAGAGGACGATCGAGTTTCGGATCTAG  -5'


A7: 5'-GATCTTACGCTGTCACCGGCCGTGGTGACTCTCCTGCTAGCTCAAAGCCT
B7: 3'-AATGCGACAGTGGCCGGCACCACTGAGAGGACGATCGAGTTTCGGA


     ATCAGCATCAACTACCGTACCGAAATCGACGGTA        -3'
     TAGTCGTAGTTGATGGCATGGCTTTAGCTGCCATCTAG    -5'
```

Man erhielt jeweils die neuen Hybridplasmide pTNF-1/6 und pTNF-1/7.

4

Vermehrung der Plasmide

## 5. Transformation der Hybridplasmide

Transformations-kompetente E.coli-Zellen wurden mit 0,1 bis 1 µg der Hybridplasmide gemäß Beispiel 1 bis 4 transformiert und auf Ampicillin enthaltenden LB-Agarplatten ausplattiert. Anschließend ließen sich Klone, die korrekt integrierte TNF-Teilsequenzen enthielten, durch Plasmidschnellanalysen identifizieren [Maniatis et al, Cold Spring Harbor Laboratory, 1982, Seite 366].

## 6. Produktion der Proteine

Die in den obigen Beispielen hergestellten Hybridplasmide werden an der Cla1-Stelle geöffnet und mit synthetisch hergestellten Signalsequenzen für die Genexpression versehen.

Mit den erhaltenen Hybridplasmiden wurden kompetente E.coli Zellen transformiert (Maniakis et al., Cold Spring Harbor Laboratory, 1982, Seite 249ff.). Der transformierte Wirtsorganismus wurde über Nacht bei 37°C in LB-Nährmedium fermentiert.

## 7. Reinigung der Proteine

1 l Fermentationsbrühe eines eine neue Substanz produzierenden E.coli-Stamms wurden 30 min bei 3000 g zentrifugiert. Der Rückstand wurde in 200 ml 0,4 M Argininhydrochlorid, 20 mM Natriumphosphat pH 8,5 aufgenommen und 30 min mit Ultraschall behandelt. Die Suspension wurde mit 6 ml 2M $MnCl_2$ versetzt und 45 min bei 3000 g zentrifugiert. Der Überstand wurde mit verdünnter $NH_3$-Lösung auf pH 8,9 gebracht und mit festem Ammoniumsulfat auf 60 % Sättigung eingestellt.

Das Proteinpräzipitat wurde in 0,2 M Argininhydrochlorid pH 7,5 suspendiert und gegen 0,4M Argininhydrochlorid pH 7,5 dialysiert. Nach 16 h wurde mit verdünnter $NH_3$-Lösung auf pH 8,5 eingestellt und auf das 5fache Volumen mit Wasser verdünnt.

Diese Lösung wurde über eine mit 0,01 M Argininpuffer pH 8,5 äquilibrierte ®R-Sepharose-Säule (Pharmacia) chromatographiert. Die Elution erfolgte mit 0,02 M Na-Phosphat, 0,06 M NaCl. Das Eluat wurde nach dem Verdünnen auf das 2,5fache über eine mit 0,02 M Na-Phosphat pH 8,0 äquilibrierte ®S-Sepharose-Säule (Pharmacia) chromatographiert. Nach Waschen der Säule mit Äquilibrierungspuffer erhielt man durch Elution mit 0,05 M Na-Phosphat, 0,1 M NaCl, 0,1 M Arginin pH 8,6 SDS-Polyacrylamidgelelektrophoretisch reines Protein.

So wurden folgende Verbindungen der Formel gemäß Anspruch 1 erhalten (n = 4, Y = Met):

A. X = ArgSerSerArgArgValThrThrAlaGlyAspArg

B. X = ArgSerTyrAlaValThrGlyArgGlyAspSerProAlaSerSerLysProArg

C.                     X                   =                   Arg-SerTyrAlaValThrGlyArgGlyAspSerProAleSerSerLysProIleSerIleAsnTyrArgThrGluIleAspGlyArg.

## 8. Cytotoxische Aktivität der neuen Polypeptide

$5 \cdot 10^3$ frisch trypsinisierte, im exponentiellen Wachstum sich befindliche Zellen wurden in 96-Loch-Platten in 150 µl komplettem Wachstumsmedium (MEM mit Earle's Salzen + 10 % FCS, Flow Laboratories, Meckenheim), ausplattiert und über Nacht bei 37°C, 5 % $CO_2$ in einer Wasserdampf-gesättigten Atmosphäre inkubiert. Die Substanzzugabe erfolgte am nächsten Tag in 25 µl komplettem Kulturmedium pro Kulturloch. Die Startkonzentration betrug 10 ng/ml; titriert wurde seriell 2fach mit Doppelbestimmungen. Folgende Kontrollen wurden auf jeder Kulturplatte mitangelegt: a) nur Kulturmedium; b) Zellen mit Kulturmedium, aber ohne Substanz; c) ein titrierter TNF-Standard bekannter biologischer Aktivität. Nach einer weiteren Inkubation von 48 h bei den oben angegebenen Bedingungen wurden die überlebenden Zellen mit einer Kristallviolettlösung (15 g Kristallviolett, 7 g NaCl 646 ml Ethanol, 172,8 ml 37 %iges Formaldehyd auf 2 l mit $H_2O$ aufgefüllt) angefärbt. Dazu wurden nach dem Ausschlagen des Kulturmediums die Zellen für 20 min mit 50 µl der Färbelösung bei Raumtemperatur versetzt. Die Kulturplatten wurden danach so lange mit Wasser gewaschen, bis alle ungebundenen Farbstoffanteile entfernt waren. Die gefärbten Zellen wurden durch Zugabe von 100 µl Färbelösung (50 % Ethanol, 0,1 % Essigsäure) lysiert und bei 540 nm photometrisch ausgewertet. Für die Substanzen ergaben sich dabei in den getesteten Zellinien folgende Aktivitäten, ausgedrückt in Einheiten pro mg Protein. Eine Einheit ist diejenige Menge an Substanz, die eine 50 %ige Lyse der behandelten Zellen induziert.

| Substanz | Biologische Aktivität in Einheiten pro mg Protein aus | | |
|---|---|---|---|
| | L-929 | WEHI-164 | MDF-7 |
| rHn-TNF | $8,2 \cdot 10^6$ | $7,9 \cdot 10^5$ | $2,6 \cdot 10^5$ |
| A | $2,0 \cdot 10^7$ | $1,3 \cdot 10^6$ | $7,5 \cdot 10^5$ |
| B | $1,8 \cdot 10^7$ | $1,6 \cdot 10^6$ | $9,5 \cdot 10^5$ |

In der Tabelle bedeuten:
L-929 eine Maus-Fibrosarkom-Zellinie,
WEHI-164 ebenfalls eine Maus-Fibrosarkom-Zellinie,
MCF-7 eine humane Mamakarzinom-Zellinie,
rHN-TNF rekombinanter humaner TNF.

**Ansprüche**

1. Proteine gekennzeichnet durch folgende Aminosäuresequenz:
Y-Val-X-(Ser)$_n$-ArgThrProSerAspLysProValAlaHisValValAlaAsnPro GlnAlaGluGlyGlnLeuGlnTrpLeuAsnArgArgAlaAsnAlaLeuLeuAlaAsnGly ValGluLeuArgAspAsnGlnLeuValValProSerGluGlyLeuTyrLeuIleTyrSer GlnValLeuPheLysGlyGlnGlyCysProSerThrHisValLeuLeuThrHisThrIle SerArgIleAlaValSerTyrGlnThrLysValAsnLeuLeuSerAlaIleLysSerPro CysGlnArgGluThrProGluGlyAlaGluAlaLysProTrpTyrGluProIleTyrLeu GlyGlyValPheGlnLeuGluLysGlyAspArgLeuSerAlaGluIleAsnArgProAsp TyrLeuAspPheAlaGluSerGlyGlnValTyrPheGlyIleIleAlaLeu,
worin
n 0, 1, 2, 3 oder 4,
X eine Teilsequenz aus der Zell-bindenden Domäne des Fibronectin-Moleküls und
Y ein Wasserstoffatom oder ein Methioninrest
sind.

2. Protein gemäß Anspruch 1, dadurch gekennzeichnet, daß X
Arg,
ArgSerArg,
ArgSerSerArgArgValThrThrAlaGlyAspArg,
ArgSerValThrGlyArgGlyAspSerProAlaArg,
TyrAlaValThrGlyArgGlyAspSerProAla,
TyrAlaValThrGlyArgGlyAspSerProIleAspGly,
ArgSerTyrAlaValThrGlyArgGlyAspSerProAlaSerSerLysProArg oder
ArgSerTyrAlaValThrGlyArgGlyAspSerProAlaSerSerLysProIleSerIleAsnTyrArgThrGluIleAspGlyArg
ist.

3. Proteine gemäß Anspruch 1, dadurch gekennzeichnet, daß Y ein Wasserstoffatom ist.

4. Verfahren zur Herstellung von Proteinen gemäß Anspruch 1, dadurch gekennzeichnet, daß man
  a. einen Vektor herstellt, der die genetische Information für die Proteine gemäß Anspruch 1 enthält,
  b. den Vektor vermehrt
  c. die für Expression nötigen Signale in den Vektor einbaut,
  d. den Vektor in einen Wirtsorganismus einbringt und
  e. den Wirtsorganismus vermehrt und das Protein isoliert.

5. Verfahren nach Anspruch 4, dadurch gekennzeichnet, daß als Wirtsorganismus E.coli verwendet wird.

6. Protein gemäß Anspruch 1 zur Verwendung bei der Bekämpfung von Krankheiten.

## TNF-cDNA

Ava I, Hind III
578 bp Fragment

|  | | 8 9 | | 157 | |
|---|---|---|---|---|---|
| | Thr | Pro Ser | | Leu STOP | Cla I / Hind III |

CGAT —— An —— ACC          CCGAGT ———————— CTG TGA —— A          amp$^R$

TA —— Bn —— TGGGGCT          CA ———————— GAC ACT ——TTCGA          pBR 322          tet$^R$

ClaI          AvaI          Ava I          HindIII          ori

T4 DNA-Ligase

amp$^R$          TNF-n

pTNF-n          tet$^R$

ori

# FIG. 1

**Konstruktion der Hybridplasmide pTNF-n**

0 291 804

FIG. 2

Konstruktion der Hybridplasmide pTNF−1/n

0 291 804

Fig.3

Fig.4